(19) **Europäisches Patentamt**

**European Patent Office**

**Office européen des brevets**

(11) Publication number: **0 269 763 B1**

(12) # EUROPEAN PATENT SPECIFICATION

(45) Date of publication of patent specification: **14.08.91**

(51) Int. Cl.⁵: **A61M 5/32**

(21) Application number: **86118046.1**

(22) Date of filing: **24.12.86**

(54) **A set of double needles for injecting liquid medicine.**

(30) Priority: **09.10.86 JP 155348/86**
**07.11.86 JP 266384/86**

(43) Date of publication of application:
**08.06.88 Bulletin 88/23**

(45) Publication of the grant of the patent:
**14.08.91 Bulletin 91/33**

(84) Designated Contracting States:
**CH DE FR GB IT LI**

(56) References cited:
**DE-A- 3 147 609**
**US-A- 3 993 079**
**US-A- 4 013 080**
**US-A- 4 511 356**
**US-A- 4 518 383**

(73) Proprietor: **HAKKO ELECTRIC MACHINE WORKS CO. LTD.**
**3055, Togura Oaza Toguramachi**
**Hanishina-gun Nagano-ken(JP)**

(72) Inventor: **Tanaka, Masataka c/o Hakko Electric Machine**
**Works Co.,Ltd 1490, Oaza Isobe Togura-machi**
**Hanishina-gun Nagano-ken(JP)**

Inventor: **Ohto, Masao**
**957, Togane**
**Togane-shi Chiba-ken(JP)**
Inventor: **Sekine, Tetsuo**
**c/o hakko Shoji Co., Ltd. 14-11, Ueno 5-chome**
**Taito-ku Tokyo(JP)**
Inventor: **Takahashi, Hiroshi**
**c/o Hakko Shoji Co.,Ltd. 14-11, Ueno 5-chome**
**Taito-ku Tokyo(JP)**
Inventor: **Maruyama Masaru c/o Hakko Electric Machine**
**Togure-machi**
**Hanishina-gun Nagano-ken(JP)**

(74) Representative: **Goddar, Heinz J., Dr. et al**
**FORRESTER & BOEHMERT Widenmayerstrasse 4/I**
**W-8000 München 22(DE)**

## Description

The invention relates to a set of double needles for injecting liquid medicine, comprising an inner needle and an outer needle into which said inner needle is inserted, wherein said inner and outer needle are fixed to bases respectively, and said base for said outer needle has a measuring mark for measuring a protruding length of said inner needle thereon.

A device known in this field is described in the US-A-3 993 079 which relates to such a set of double needles.

The set of needles described in this patent has the disadvantage that always the same part of the human body is punctured by the inner needle. In addition there are no means to spread the liquid medicine to different parts of a predetermined area by using only a single puncturing.

Accordingly, it is an object of the invention to provide a set of double needles for injecting liquid medicine by which liquid medicine is injected into a plurality of affected parts in a single puncturing thereof in a predetermined area thereby to a avoid the increase of a pain for a patient by using different ways in which the inner needle is puncturing said affected parts.

It is further desirable to provide a set of double needles by which an injecting point can be varied in a predetermined way.

According to the object of this invention the inner needle is provided with a curved portion at its tip, said curved portion being straightened within said outer needle when retracted in a predetermined length L thereof, said base for said inner needle is provided with projections thereon, said base for said outer needle is provided with two grooves thereon into which said projections are engaged, and the size of the groove which is provided at the back side of said base for said outer needle is slightly greater than that of the groove which is provided at the front side of said base for said outer needle.

It is advantageous that said base for said outer needle is transparent and has a standard mark for angle rotation of said base for said inner needle.

Further it is recommended that each of said bases for said inner and outer needles comprises a square portion to allow the rotation of each by 90 degrees.

The invention will be described in detail in accordance with the following drawings, wherein

Fig.1 to 3
are cross-sectional views illustrating a relation between an inner and an outer needle according to the invention,

Fig. 4
is an explanatory view showing a covering area of a single puncturing of a set of double needles according to the invention,

Fig. 5A
is a cross-sectional view illustrating a base fixing an outer needle thereto according to the invention,

Fig. 5B
is a perspective view illustrating a base fixing an inner needle thereto according to the invention,

Fig. 6A
is a perspective view illustrating a set of double needles for injecting liquid medicine in the preferred embodiment according to the invention, and

Fig. 6B and 6C
are cross-sectional views each illustrating a relation of bases for inner and outer needles in the preferred embodiment according to the invention.

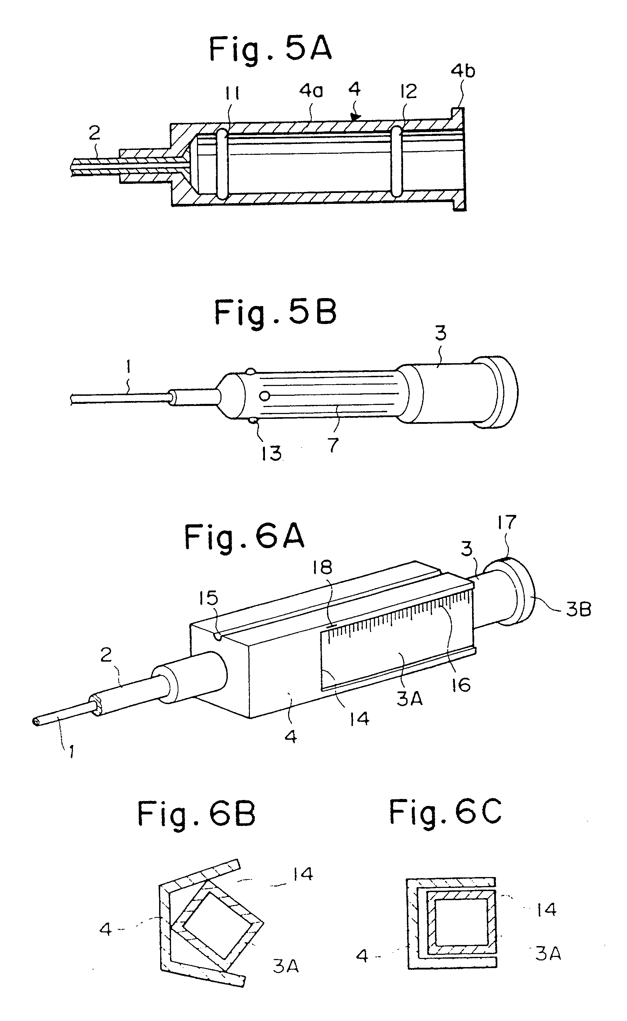

In Fig. 5A und 5B, the two parts of the set of double needles for injecting liquid medicine are shown. There are provided an inner needle 1 having a curved portion at the tip thereof and an outer needle 2 into which the inner needle 1 is inserted. The inner and outer needle 1 and 2 are provided with bevels 1a and 2a respectively and the inner needle 1 is longer than the outer needle 2 so that the curved portion of the inner needle 1 protrudes from the outer needle 2 as described later in more detail when the inner needle 1 is fully inserted into the outer needle 2.

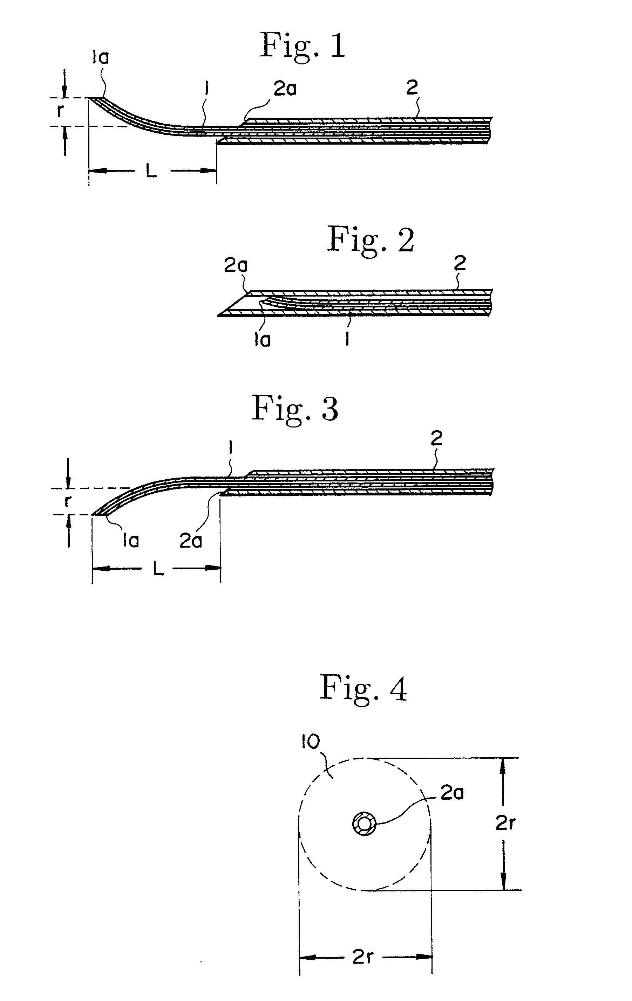

Further the inner and outer needles 1 and 2 are fixed to bases 3 and 4 respectively. The base 4 for the outer needle 2 is transparent in a prefered embodiment and comprises a main body 4a having a measuring mark thereon and an end portion having a standard mark thereon. On the other hand the base 3 for the inner needle 1 is provided with an angle mark 7 thereon. In operation the bevel 1a for the inner needle 1 is positioned at the distance r from the center line because the inner needle 1 has the curved portion at the top thereof when the needle 1 protrudes from the bevel 2a for the outer needle 2 in a predermined length L of, for instance 25 mm as shown in Fig. 1.

The inner needle 1 is straightened in the outer needle 2 in accordance with the resilient deformation thereof when the bevel 1a of the inner needle 1 is retracted into the outer needle 2 as shown in Fig. 2. At this stage, the inner needle 1 is rotated by 180 degrees in accordance with the angle mark 7 on the base 3 and the standard mark on the end portion of the base 4. The bevel 1a positioned at the distance r in opposite direction from the center line due to the resilience thereof when the inner needle 1 protrudes from the bevels 2a for the outer needle 2 in a predetermined length L mentioned above as shown in Fig. 3. The protruding length L is adjusted by determining the front end portion 3a for the base 3 in accordance with the measuring mark.

It is understood form the explanation as shown in Fig. 4 that liquid medicine is projected to cover a predetermined area 10 having a diameter 2r the center of which is indicated at the bevel 2a for the outer needle 2.

In Fig. 5A and 5B there are shown the two parts of a set of double needle for injecting liquid medicine according to the invention with special reference to grooves in the base 4 of the outer needle 2 and projections 13 on the base 3 for the inner needle 1.

The base 3 is provided with projections 13 thereon each of which is deformed resiliently, while the base 4 is provided with grooves 11 and 12 on the inner surface thereof. In Fig. 5A and 5B like reference numerals indicate like parts as in Fig. 6A.

In operation the base 3 is positioned inside the base 4 such that the inner needle 1 is inserted into the outer needle 2. At this stage the projections 13 are engaged into the groove 12 and the base 3 is rotated by a predetermined angle in accordance with the standard mark on the end portion and the angle mark 7 on the base 3. At the next stage, the base 3 is pushed forward to the projections 13 are compressed, whereby the base 3 is moved in the slidable manner inside the base 4 and that the projections 13 are engaged into the groove 11 thereby the base 3 is stopped while the inner needle 1 protrudes from the bevel 2a of the outer needle 2 in a predetermined length L as mentioned before. The size of groove 12 is slightly greater than that of groove 11, such that the force of engaging the projections 13 into the groove 11 is slightly greater than that of engaging the projections 13 into the groove 12 so that the handling of the double needle becomes easier.

In Fig. 6A to 6C, there is shown a set of double needles in another embodiment according to the invention wherein a base 4 for an outer needle 2 is of a square having an opening portion 14 at the side thereof, a slit 15 to be utilized as a standard mark for the rotation at the other side thereof and a mark 18 to be utilized as a standard point. On the other hand, the base 3 for the inner needle 1 comprises a square portion 3a and a circular portion 3b to which a syringe (not shown) is connected. A measuring mark 16 is provided on a square portion 3a and a standard mark 17 is provided on the circular portion 3b so that the corresponding relation between the inner and outer needles 1 and 2 is known in accordance with the relative positions of the mark 16 and 17 in regard to the slit 15 and mark 18.

In operation, the base 3 is pulled back such that the top as thereof is positioned at the opening portion 14 thereby the base 3 is rotated as shown in Fig. 6B. In rotation of the base 3, the base 4 is deformable so that the base 3 can be easily stopped after the rotation of 90, 180 or 270 degrees. Thereafter the base 3 is pushed forward so that the inner needle 1 protrudes from the bevel 2a of the outer needle 2 in a predetermined length by checking the measurung mark 16 in regard to the mark 18.

## Claims

1. A set of double needles for injecting liquid medicine, comprising an inner needle (1) and an outer needle (2) into which said inner needle (1) is inserted, wherein said inner and outer needles are fixed to bases (3, 4) respectively, and said base (4) for said outer needle (2) has a measuring mark for measuring a protruding length of said inner needle (1) thereon,

   characterized in that

   - the inner needle (1) is provided with a curved portion at its tip, said curved portion being straightened within said outer needle (2) when retracted in a predetermined length L thereof,
   - said base (3) for said inner needle (1) is provided with projections (13) thereon,
   - said base (4) for said outer needle (2) is provided with two circumferential grooves (11, 12) thereon into which said projections (13) are engaged, and
   - the size of the groove (12) which is provided at the back side of said base (4) for said outer needle

   (2) is slightly greater than that of the groove (11) Which is provided at the front side of said base (4) for said outer needle (2).

2. A set of double needles for injecting liquid medicine according to claim 1, characterized in that said base (4) for said outer needle (2) is transparent and has a standard mark for angle rotation of said base (3) for said inner needle (1), and said base (3) for said inner needle (1) has an angle mark (7) for angle rotation of said base (3) for said inner needle (1).

3. A set of double needles for injecting liquid medicine according to one of the preceding claims, characterized in that each of said bases (3, 4) for said inner (1) and outer (2) needles comprises a square portion to allow the rotation of each by 90 degrees.

## Revendications

**EP 0 269 763 B1**

1. Jeu d'aiguille double pour l'injection de médicament liquide, comprenant une aiguille intérieure (1) et une aiguille extérieure (2) dans laquelle est insérée ladite aiguille intérieure (1), jeu dans lequel lesdites aiguilles intérieure et extérieure sont fixées à des bases respectives (3, 4), et ladite base (4) de l'aiguille extérieure (2) comporte une repère de mesure pour mesurer une longueur de dépassement de l'aiguille intérieure (1), caractérisé par le fait que
   - l'aiguille intérieure (1) présente une partie courbe a son extrémité, ladite partie courbe étant redressée à l'intérieur de l'aiguille extérieure (2) lorsqu' elle y est rentrée sur une longueur prédéterminée L,
   - ladite base (3) de l'aiguille intérieure (1) comporte des saillies (13),
   - ladite base (4) de l'aiguille extérieure (2) présente deux gorges circonférentielles (11, 12) dans lesquelles sont engagées lesdites saillies (13), et
   - la dimension de la gorge (12) qui se trouve à l'arrière de ladite base (4) de l'aiguille extérieure (2) est un peu plus grande que celle de la gorge (11) qui se trouve à l'avant de ladite base (4) de l'aiguille extérieure (2).

2. Jeu d'aiguille double pour l'injection de médicament liquide selon la revendication 1, caractérisé par le fait que ladite base (4) de l'aiguille extérieure (2) est transparente et porte une marque de référence pour déterminer l'angle de rotation de ladite base (3) de l'aiguille intérieure (1), et ladite base (3) de l'aiguille intérieure (1) porte une repère d'angle (7) pour déterminer l'angle de rotation de ladite base (3) de l'aiguille intérieure (1).

3. Jeu d'aiguille double pour l'injection de médicament liquide selon l'une des revendications précédentes, caractérisé par le fait que chacune desdites bases (3, 4) des aiguilles intérieure (1) et extérieure (2) comprend une partie carrée pour permettre la rotation de chacune de 90 degrés.

**Patentansprüche**

1. Doppelnadel-Set zum Injizieren eines flüssigen Medikaments, mit einer Innennadel (1) und einer Außennadel, (2) in die besagte Innennadel (1) eingeschoben ist, wobei besagte Innen- und Außennadeln jeweils an Grundkörpern (3,4) befestigt sind und besagter Grundkörper (4) für besagte Außennadel (2) eine Meßmarkierung zum Abmessen einer herausragenden Länge besagter Innennadel (1) aufweist, dadurch gekennzeichnet, daß
   - die Innennadel (1) mit einem gebogenen Abschnitt an ihrer Spitze versehen ist, wobei besagtergebogener Abschnitt innerhalb besagter Außennadel (2) gerade gebogen wird, wenn er in einer vorbestimmten Länge L zurückgezogen wird,
   - besagter Grundkörper (3) für besagte Innennadel (1) mit Vorsprüngen (13) versehen ist,
   - besagter Grundkörper (4) für besagte Außennadel (2) mit zwei Umfangsnuten (11,12) versehen ist, in die besagte Vorsprünge (13) eingreifen, und
   - die Größe der Nut (12), die an der hinteren Seite besagten Grundkörpers (4) für besagte Außennadel (2) vorgesehen ist, etwas größer ist als diejenige der Nut (11), die an der vorderen Seite besagten Grundkörpers (4) für besagte Außennadel (2) vorgesehen ist.

2. Doppelnadel-Set zum Injizieren eines flüssigen Medikaments nach Anspruch 1, dadurch gekennzeichnet, daß besagter Grundkörper (4) für besagte Außennadel (2) durchscheinend ist und eine Standardmarkierung für die Winkeldrehung besagten Grundkörpers (3) für besagte Innennadel (1) aufweist, und daß besagter Grundkörper (3) für besagte Innennadel (1) eine Winkelmarkierung (7) für die Winkeldrehung besagten Grundkörpers (3) für besagte Innennadel (1) aufweist.

3. Doppelnadel-Set zum Injizieren eines flüssigen Medikaments nach einem der vorangehenden Ansprüche, dadurch gekennzeichnet, daß jeder besagte Grundkörper (3, 4) für besagte Innen- (1) und Außennadel (2) einen quadratischen Abschnitt umfaßt, um die Drehung eines jeden um 90° zu ermöglichen.

4

## Fig. 1

## Fig. 2

## Fig. 3

## Fig. 4

## Fig. 5A

## Fig. 5B

## Fig. 6A

## Fig. 6B

## Fig. 6C